# EUROPEAN PATENT APPLICATION

(11) **EP 1 645 224 A1**
(43) Date of publication of application: **12.04.2006**
(21) Application number: 05380206.2
(22) Date of filing: 23.09.2005
(51) Int. Cl.: A61B 5/00, A61B 5/0408

(54) **Device for the continuous and similtaneous monitoring of physiological parameters of a patient, in particular, cardiological parameters**

(30) Priority: 07.10.2004 ES 200402387 P
(71) Applicant: Mula Galera, Pilar, 08950 Esplugues de Llobregat (ES); Vidal Fortuny, Jordi, 08195 Sant Cugat del Valles (ES)
(72) Inventor: Mula Galera, Pilar, 08950 Esplugues de Llobregat (ES); Vidal Fortuny, Jordi, 08195 Sant Cugat del Valles (ES)
(74) Representative: SUGRANES - VERDONCES - FERREGÜELA

(57) **Abstract**

Device for the continuous and simultaneous monitoring of multiple physiological parameters of a patient, especially for a patient in surgery, a patient in a postoperative reanimation room or under medical observation, which comprises many sensors (1) connected to a radio-frequency signal transmitter (2), which communicates with a receiver (3). At least two of the sensors are positioned adjacent and connected to one another via a single cable (4), one end of the cable being connected to the radio-frequency signal transmitter.

## Description

### Technical field of the invention

The present invention relates to a device for the continuous and simultaneous monitoring of a patient's physiological parameters, particularly cardiological parameters, and especially for a patient in surgery, a patient in a postoperative reanimation room or under medical observation, comprising many sensors connected to a radio-frequency signal transmitter, which itself communicates with a receiver located inside a piece of monitoring equipment.

### Background of the invention

It is known that during surgery or when the patient is under observation or in a postoperative room, various physiological parameters must be monitored and/or checked providing information on the patient's condition at all times. Said physiological parameters are usually measured as electric parameters detected by electrodes and transmitted via cables to a piece of monitoring equipment. Thus, in the field of anaesthesia and in certain medical examinations, the cabling between the different sensors (cardiac electrodes, if we are carrying out an electrocardiogram, pulse oximeter, etc.), located on the patient's body and the monitoring equipment, make this indispensable. It should be emphasized here that if the patient is being monitored within the surgical area, said cabling can hinder the mobility of the nurse and other medical staff, and may lead to monitoring errors under the control of the nurse if, for example, the patient is accidentally disconnected from said cables. Although the monitoring is therefore acceptable, it makes it awkward for the medical staff and for the patient himself, which hinders them in carrying out their various tasks inside and outside the operating theatre, and may also constitute a possible danger for the patient due to the errors that occur in said monitoring.

In order to overcome the presence of such a high number of cables between the patients and the monitoring equipment, systems have been devised based on the transmission of data via electromagnetic waves, more specifically via radio frequencies. One of the first patents to disclose a system of this type is patent US 5,704,351, in which a radio-frequency transmitter is used to measure the analogue signals from different sensors that check physiological parameters, convert them into digital signals and transmit them as radio-frequency signals to a piece of monitoring receiving equipment located some distance from the transmitter. This patent thus eliminates the cables that go from the patient direct to the monitoring equipment.

In the same way as in patent US 5,704,351, patent US 6,749,566 discloses a network environment for a patient which comprises at least two devices connected to the patient, each of these devices also comprising at least one sensor for detecting the patient's parameters; and at least one radio-frequency transmitter able to communicate with a monitor; and one processor monitor for the patient, able to receive the transmitter's signal via cable-free technology. The monitor is configured so that it can display onscreen the data of at least the two devices connected to the patient; interpret the physiological data; and synchronize the operation of each of the devices connected to the patient with the other device. This environment applies to electrocardiograms, electroencephalograms, etc. The invention provides an exhaustive account of how the data is interpreted, checked and processed via the monitor's processor. Although, in line with the figures used in this environment, the number of cables has been reduced, there are still many cables between the patient and the radio-frequency transmitters, which are a hindrance and can be uncomfortable for the patient.

In an attempt to reduce the cabling near the patient to a minimum, patent US 5,511,553 discloses a cable-free electrode structure, which includes a non-conductive strip long and wide enough to contain a signal transmitter on one side and a plurality of pairs of conductive elements on the other side. The transmission between a transmitting device located on the strip, and a receiving device, is done by radio frequency. However, a disadvantage of said electrode structure is that it is not easily adaptable to different patients, since various measures must be taken so that the sensors are located in the correct areas to make the measurements. In the end, it is better to resort to the conventional techniques with cables between the patient and the radio-frequency transmitter.

Lastly, another of the problems with systems or devices for the continuous and simultaneous monitoring of many physiological parameters, which are wireless or cable-free, is that they are expensive due to the fact that the signals generated are usually complex and fidelity systems must be developed for them which are also very complex themselves.

With the aim of resolving the abovementioned problems, the present invention offers inventive solutions which make the device adaptable, less complex and, therefore, cheaper, compared to the devices already known.

### Explanation of the invention

The device for the continuous and simultaneous monitoring of a patient's multiple physiological parameters, particularly cardiological parameters, being the object of the invention, is characterized in that at least two of the sensors it contains are positioned adjacent and connected to one another via a single cable, one end of such cable being connected to the radio-frequency signal transmitter.

According to another characteristic of the invention, the sensors are attached to the cable via a connector comprised of:
a) a lower plate which comprises the sensor, means for fastening the sensor, and a cable output;
b) an upper plate with a cable input from a connector with adjacent sensor; and
c) an intermediate area wherein some interconnection means are provided between the upper and lower plates.

The device for the continuous and simultaneous monitoring of multiple physiological parameters is also characterized in that the interconnection means between the upper and lower plates comprise a rotating axle.

According to another characteristic of the device of the invention, the connecting cable between sensors is an extendable cable.

The device for the continuous and simultaneous monitoring of multiple physiological parameters is also characterized in that it comprises at least one sensor which is branch-connected to the transmitter.

The device for the continuous and simultaneous monitoring of multiple physiological parameters according to the invention is characterized in that it comprises at least one sensor branch-connected to one or more of the sensors.

### Brief description of the drawings

The attached drawings show, as a non-restrictive example, a device for the continuous and simultaneous monitoring of multiple physiological parameters. In said drawings:
- Figs. 1 to 4: correspond to plan views of different embodiments of the device according to the invention;
- Fig. 5: shows an elevational view of a connector built in to the device, which consists of a lower plate for housing a sensor, an upper plate and an intermediate area with interconnection means between the two upper and lower plates;
- Fig. 6: is a diagrammatical view of the lower plate of the connector in Fig. 5;
- Fig. 7: corresponds to a diagrammatical view of the upper part (upper plate) of the same connector in Fig. 5; and
- Fig.8: shows the device for the continuous and simultaneous monitoring of multiple physiological parameters, applied to a patient.

### Detailed description of the drawings

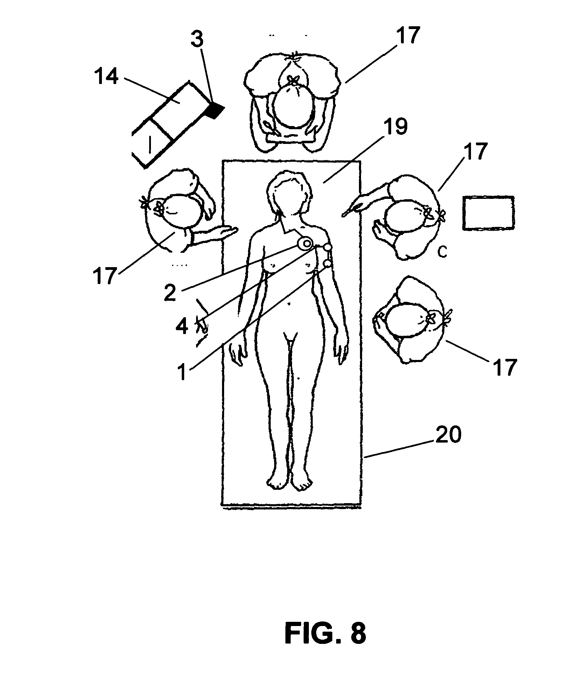

As can be seen from the attached drawings, the device for the continuous and simultaneous monitoring of multiple physiological parameters, particularly cardiological ones according to the invention, is suitable for carrying out surgery on a patient 19 with maximum mobility for the medical staff 17 and without the hindrance caused by cables. And, therefore, in accordance with Fig. 8, in wherein a trolley bed 20 is diagrammatically represented with a patient 19 being operated on by specialized staff 17, it can be seen that the only cable present is cable 4, which picks up the signals from some sensors 1, used to detect some of the relevant vital signs at any time. This cable 4 rests on the patient 19 himself. The physiological parameters are determined via the sensors 1 placed on the patient 19 and are transmitted via radio-frequency signals emitted by a transmitter 2, and represented in Fig. 8 by an arrow, to a universal receiver 3 positioned in an adjoining piece of monitoring equipment 14.

With the aim of simplifying the cabling between the patient 19 and the monitoring equipment 14, and to reduce the number of cables on the patient, the device, according to the invention, comprises at least two sensors 1 positioned adjacent and connected to one another via a single cable 4. The end of the cable without sensors is connected to the radio-frequency signal transmitter 2.

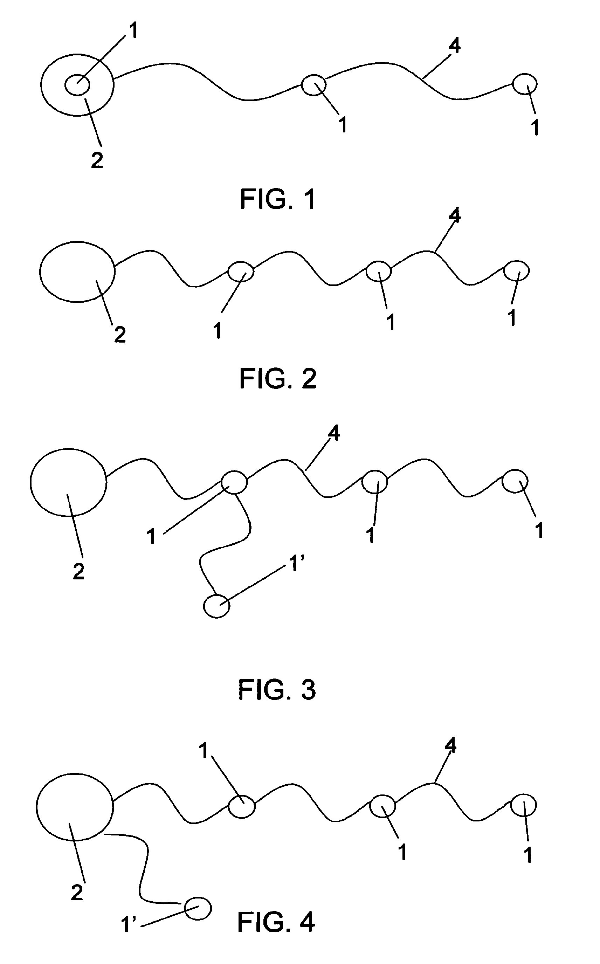

In order to appreciate the different, effective possible layouts, in accordance with the invention, Figs. 1 to 4 represent various sensors 1 connected via cable 4 which will be placed on the patient 19. Both Fig. 1 and Fig. 2 comprise three sensors 1 connected to one another via a single cable 4 and to a radio-frequency signal transmitter 2. More specifically, Fig. 1 shows the possibility that at least one of the sensors 1 is located inside said transmitter 2, the transmitter 2 thus also being in contact with the patient 19. In comparison, in Fig. 2, the radio-frequency signal transmitter 2 contains no sensor 1 inside. In this latter case, when applied to a patient 19, the sensors 1 are placed on the thorax of the patient 19 and the transmitter 2 is positioned on the trolley bed 20 beside the patient 19, and is connected to the sensors 1 via the free end of the cable 4.

Figs. 3 and 4 show other embodiments of the device for the continuous and simultaneous monitoring of multiple physiological parameters, particularly cardiological ones. These figures consider the possibility that in addition to at least two of the sensors 1 being adjacent and connected to one another via a single cable 4, there is also a sensor 1' branch-connected, in one case to the transmitter 2 (Fig. 4), and in another case, to one of the sensors 1 (Fig. 3).

Obviously, although they are not represented in Figs. 3 and 4, inside the radio-frequency signal transmitter 2, there can also be a sensor 1, the same as in the case shown in Fig. 1. In this latter case, the sensor 1-transmitter 2 array must also be placed on the patient 19.

Similarly, although they are not represented in Figs. 3 and 4, there may be one or more than one branch sensors 1', also adjacent and joined to one another via a single cable 4.

The radio-frequency signal transmitter 2 receives the electrical signals from the sensors 1 and can amplify, condition and convert them into digital signals. Said digital signals containing the information regarding the physiological parameters will be transmitted in code via radio frequency to a receiver 3 located in the standard monitoring equipment 14, in the operating theatre, postoperative room or observation room. The transmitter 2 has some means of power supply, such as batteries, so that it can carry out the whole process of acquisition and transmission of data.

In order to avoid errors in data reception, there is an encoding process so that each transmitter 2 contains a unique serial number which differentiates it from the other transmitters 2. By means of a programming process, the receiver 3 is programmed to recognize only the data from the transmitter 2 selected.

The radio-frequency signal received is converted into an analogue signal with voltage values suitable for handling. This signal is then sent to a signal regeneration circuit so that it can convert it into voltage and current values suitable for viewing on screen, not represented, on the monitoring equipment 14.

In order to avoid interference between devices for the continuous and simultaneous monitoring of multiple physiological parameters located close by, the transmit signal is preferably very low power, so that its range is only a few metres. Similarly, a given transmitter 2 and a given receiver 3 can be designed to work in a set frequency band, different from another transmitter 2-receiver 3 pair.

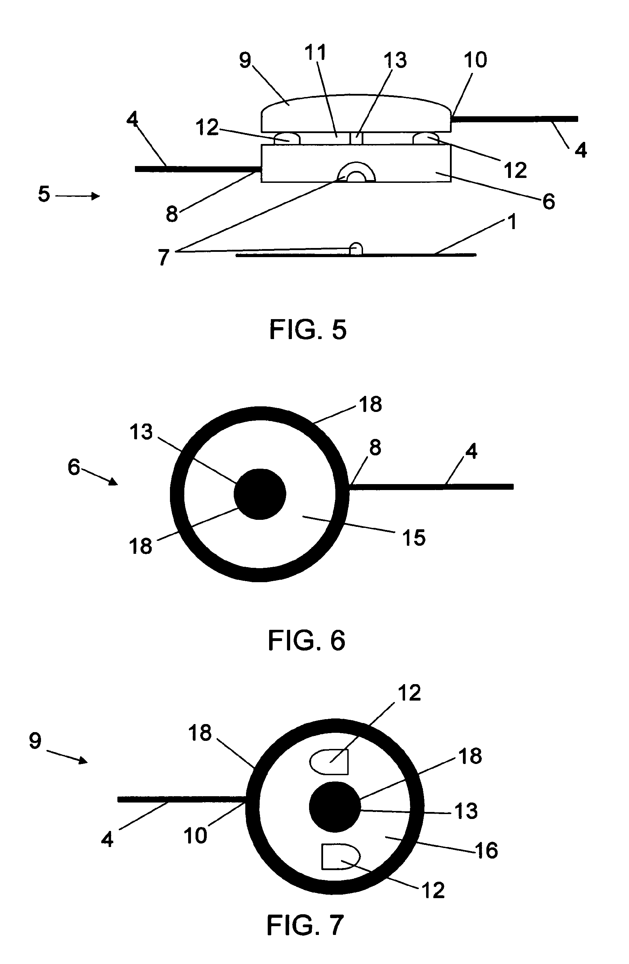

Another very beneficial aspect of the device according to the invention is that, in order to make positioning of the sensors 1 easier and more versatile, they are attached to the cable 4 via a connector 5 comprised of:
a) A lower plate 6 which, in accordance with Fig. 6, comprises a surface of conductive material 15 for transmitting the signal from a sensor 1; means for fastening the sensor 7 which, in Fig. 5, are represented by a slot-stud joint; and a cable output 8.
b) An upper plate 9, whose plan view is represented in Fig. 7 and which also comprises a conductive surface 16; and a cable input 10 from a connector 5 with adjacent sensor.
c) An intermediate area 11 in which there are some interconnection means (12, 13) which, in the case represented, according to Figs. 5 and 7, are comprised of an axle 13, which is preferably rotating, and by some pieces of conductive material 12.

In accordance with the different parts of the connector 5 represented in Figs. 6 and 7, the structure forming the lower 6 and upper 9 plates is comprised of material with insulating properties 18.

Provision is also made for the connecting cable 4 between sensors 1 to be preferably of an extendable material.

The join between the rotating axle 13 and the extendable cable 4 thus allows the device according to the invention to be adapted to different positions of the patient 19 or to different patients, for example, to adult men, children or women. There is therefore no need to manufacture a great number of devices of varying magnitude, because with a few different-sized models, all possible patients 19 can be provided for.

With a device according to the invention, the twelve cardiac derivations, known as the precordial derivations, can be monitored.

Said signals derive from the use of three electrodes or sensors 1, called R, which corresponds to the right arm; L, which corresponds to the left arm; and F, which corresponds to the left leg. The following measurements are obtained from said sensors (R, L, F):
- I: difference of signals between L and R.
- II: difference of signals between F and R.
- III: difference of signals between F and L.

By using these sensors 1 (R, L, F), the following average values can also be obtained:
- aVR: measurement between value R and the average of the L and F values.
- aVL: measurement between value L and the average of the R and F values.
- aVF: measurement between value F and the average of the R and L values.

Lastly, by using six more electrodes or sensors 1, we can obtain the six remaining measurements of the precordial derivations, known by those skilled in the art as:
- V1: fourth right intercostal space, right parasternal line.
- V2: fourth left intercostal space, left parasternal line.
- V3. symmetrical between V2 and V4.
- V4: fifth left intercostal space, mid-clavicular line.
- V5: fifth left intercostal space, anterior axillary line.
- V6: fifth left intercostal space, mid-axillary line.

It should also be emphasized that, using the device according to the invention, the movement of the thorax can also be measured simultaneously, which is another of the physiological parameters to be monitored. This is possible because with the electrical signals (difference in voltage) detected by the sensors 1, a graph can be drawn, which represents the frequency and rhythm of breathing.

Although in the case represented by Fig. 8, the patient 19 is positioned on a trolley bed 20, for example an operating table, said bed 20 being situated close to a piece of monitoring equipment 14, the device for the continuous and simultaneous monitoring of physiological parameters of a patient according to the invention, can also be used for patients 19 to whom sensors 1 as detailed in the invention are applied for a long period of time (during sleep, 24 hours, 48 hours, etc.), generally in their own home, and which transmit the signals detected to a holter receiver 3. In this case, the signals received by the holter receiver 3 will be transmitted to a data control centre by wireless connection, for example, via a Wi-Fi, GSM or GPRS network. In this way, the data stored in the holter memory are compiled periodically in the control centre, together with any alarms detected concerning the cardiac dysfunctions that are likely to be identified as critical and can, if necessary, act with the maximum possible speed.

The benefits of the device for the continuous and simultaneous monitoring of physiological parameters of a patient according to the invention, are a direct consequence of its composition. Thus, due to the minimized number of cables for the sensors 1, the device is economical, easy for medical staff to use and comfortable both for the patient 19 and such staff, who do not have to work at the side of a bed 20 which is communicating with a piece of monitoring equipment 14 with many cables. In the device according to the invention, the only cable 4 that exists is the one which connects the different connectors 5, which include the sensors 1, and the end of this cable 4, which is joined to a transmitter 2, said transmitter 2 itself being situated on the bed 20, or in the case of a transmitter 2 with a sensor 1 inside, on the corresponding part of the patient's 19 thorax. In addition, the device is designed to be compatible with the receivers 3 which are most often used to form part of the monitoring equipment 14 in medical centres or hospitals, for which there is no great need to adapt the monitoring equipment 14 commonly used in said centres.

A provision is also made for most of the parts of the device, more specifically, for the cable 4, to be disposable, which implies greater hygiene in general for the device.

## Claims

1. Device for the continuous and simultaneous monitoring of a patient's physiological parameters, particularly cardiological ones, and especially for a patient in surgery, a patient in a postoperative reanimation room or under medical observation, which comprises many sensors (1) connected to a radio-frequency signal transmitter (2), which itself communicates with a receiver (3), **characterized in that** at least two of the sensors are positioned adjacent and connected to one another via a single cable (4), one end of the cable being connected to the radio-frequency signal transmitter.

2. Device for the continuous and simultaneous monitoring of multiple physiological parameters according to claim 1, **characterized in that** the sensors (1) are attached to the cable (4) via a connector (5) comprised of:
a) a lower plate (6) which comprises the sensor, means for fastening the sensor (7) and a cable output (8);
b) an upper plate (9) with a cable input (10) from a connector with adjacent sensor; and
c) an intermediate area (11) in which there are interconnection means (12) between the upper and lower plates.

3. Device for the continuous and simultaneous monitoring of multiple physiological parameters according to claim 2, **characterized in that** the interconnection means (12) between the upper (9) and lower (6) plates comprise a rotating axle (13).

4. Device for the continuous and simultaneous monitoring of multiple physiological parameters according to any of the previous claims, **characterized in that** the connecting cable (4) between sensors is an extendable cable.

5. Device for the continuous and simultaneous monitoring of multiple physiological parameters according to any of the previous claims, **characterized in that** it comprises at least one sensor (1) branch-connected to the transmitter (2).

6. Device for the continuous and simultaneous monitoring of multiple physiological parameters according to any of the previous claims, **characterized in that** it comprises at least one sensor (1) branch-connected to one or more of the sensors.
